# EUROPEAN PATENT APPLICATION

(11) **EP 0 588 756 A2**
(43) Date of publication of application: **23.03.1994**
(21) Application number: 93650031.3
(22) Date of filing: 17.09.1993
(51) Int. Cl.: A61F 2/52

(54) **Attachment of a breast prosthesis**

(30) Priority: 17.09.1992 IE 922668
(71) Applicant: EUROMAIL LIMITED, Tallaght, Dublin 24 (IE)
(72) Inventor: Brogan, Benedict, Lucan, County Dublin (IE)
(74) Representative: Weldon, Michael James

(57) **Abstract**

A fastener (1) has an elastic film (2) of semi-permeable polymer material. Adhesive (5) is adhered in a diagonal grid pattern to the rear surface (3) of the film (2) and adhesive (6) is applied in a transverse striped pattern to a front surface (4) of the film (2). Both adhesives have a high shear strength and a low peel resistance. The rear surface of the film (2) adheres to the user's skin, and the front surface removably adheres to the rear surface of any conventional prosthesis. A fastener (1) may be used for up to three days and is then disposable. The fastener is relatively inexpensive to produce and provides versatility for a user in that she may use any different conventional prosthesis with a fastener. Because only the thickness of the film (2) separates the prosthesis (10) from the skin, the prosthesis feels natural to the user.

## Description

The invention relates to the attachment of a breast prosthesis to the chest of a user. In particular, the invention relates to a breast prosthesis fastener having means for adhering to a user's skin and means for attachment to a prosthesis to hold the prosthesis in place.

Following a mastectomy operation, the loss of a breast is an extremely difficult matter for a woman to come to terms with. It is, therefore, extremely important that the breast prosthesis which she uses appears and feels as natural as possible. A very large amount of work has been carried out in recent years in the improvement of the composition of breast prostheses and most now comprise a two-component cross-linked silicone gel composition. The prosthesis normally has a skin of polyurethane or polyethylene material enclosing the silicone rubber.

While improvements have been made in the manner in which the prosthesis is manufactured, major problems still exist arising from the manner in which the prosthesis is worn by the user. Conventionally, the prosthesis is supported within the user's brassiere. This arrangement suffers from the major disadvantage that because the prosthesis is not bonded to the skin, psychological problems can arise. This is because the prosthesis does not feel "natural" to the woman and because it is not secure, thus leading to a lack of confidence.

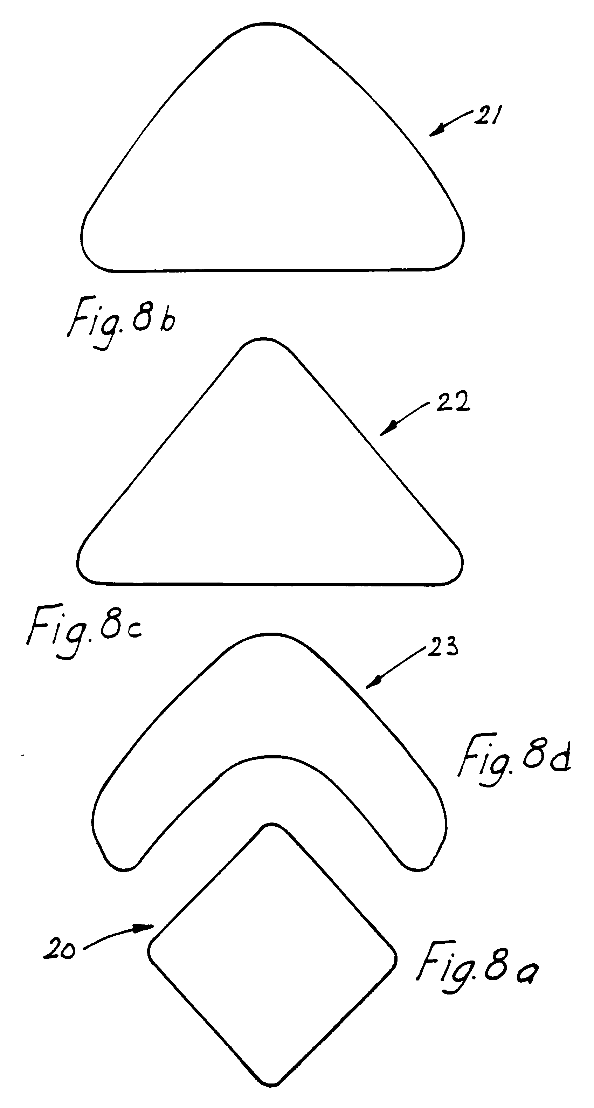

In recent years, various developments have been made to overcome the above problems. In British Patent Specification No. GB-A-2202745 (Craig Medical Products Limited), a prosthesis arrangement is shown comprising a prosthesis having one unit of a "hook and loop" fastener secured to its rear surface. There is also a separate pad, which has on one side the other unit of the "hook and loop" fastener and on the other side adhesive for attachment to the skin. As is clear from, for example Fig. 5 of this specification, there is a large gap between the prosthesis and the user's skin because of the thickness of the pad and fastener. Accordingly it appears that the prosthesis would not feel very natural to the user. Further, the prosthesis would be more expensive to produce than a conventional prosthesis because it is necessary to bond a unit of the fastener to the rear surface during manufacture. In addition, there is little versatility in use of the prosthesis because it can only be used fixed in position with the pad as otherwise the fastener would irritate the skin.

United States Patent Specification No. 4100621 discloses an arrangement whereby hook type fasteners are arranged to extend outwardly from a prosthesis. These fasteners may be used for securing the prosthesis to a nightgown, again hardly mirroring the natural situation.

European Patent Specification No. EP-A1-0392960 (Coloplast) discloses a prosthesis having special ledges in the rear of it to which various units of hook and loop fasteners are bonded. There are separate pads having engaging units to complete a fastener on one side and adhesive on the other side for securing the prosthesis to the skin. Because of the use of ledges, the problem of there being a gap between the periphery of the prosthesis and the skin are overcome to some extent. However, the disadvantages of the requirements for an expensively produced and dedicated prosthesis are not overcome and there appears to be little versatility in use of the prosthesis.

U.S. Patent Specification No. 4426742 (Beiersdorf AG) discloses a prosthesis having a recess in which there is an adaptor type moulding retained, the moulding being adhered to the skin. The prosthesis is rotatable about the moulding. Again, this prosthesis would be expensive to produce because of the special recess which is required, and the connector which comprises a special adaptor for fitting into the prosthesis would also be expensive to produce. Similar comments apply to the arrangement disclosed in European Patent Specification No. EP-A1-0542119 (Amoena) in which there is a special insert within the prosthesis extending around the rear periphery of the prosthesis, this insert being bonded to the skin by adhesive.

German Patent Specification No. DE-A-27 42 394 (Lohse) discloses an arrangement whereby six fasteners extend radially from the prosthesis for bonding to the skin. The rear of the prosthesis must be constructed specially to connect to these fasteners and because the fasteners are visible, they would do little to improve the user's confidence.

In general, despite the ingenuity and complexity of the presently available arrangements for attachment of prostheses, problems still remain. It must not be forgotten that breast prostheses are largely a cosmetic product and are mainly medical for a psychological viewpoint.

The primary objects of the present invention are as follows:-
1. to provide for adhering a prosthesis to the body in a manner which more closely resembles the natural situation, so that awareness of the prosthesis by the user is considerably reduced and her confidence is improved,
2. to considerably reduce the costs involved, and
3. to provide for versatility in choice and use of prostheses by the user.

The invention is characterised in that:-
the body portion comprises a film shaped to conform to at least portion of the rear surface of a breast prosthesis, the film having a skin-engaging rear surface and a prosthesis-engaging front surface;
the rear surface has a skin-compatible adhesive of high shear strength and low peel resistance; and
the front surface has a plastics-compatible adhesive of high shear strength and low peel resistance for removable attachment of the film to the rear surface of the prosthesis.

Because adhesives are used on both sides of the film, the prosthesis may be bonded closely to the user's skin to more closely resemble the natural feel to a user. Further, because the fastener adheres in a removable way to the rear surface of the prosthesis, most conventional prostheses would be suitable for use with the fastener and the costs involved in purchasing a special, expensively produced, prosthesis are avoided. The only additional costs to the user are the costs of the fastener. In addition, because most conventional prostheses can be used with the fastener, the user may choose from a number of different prostheses which she may own and thus there is improved versatility.

The invention is undoubtedly simple, probably because a different approach to the problem has been taken. One could almost suggest that in the past there has been too much complexity and too little appreciation of the users requirements. Undoubtedly, the simplicity of the invention is one of its major advantages. It could almost be said that the use of adhesives on both sides of a film is directly contrary to conventional wisdom of experts in this field, and hence the elaborate prior art arrangements.

In one embodiment, the film is of an elastic material having an elasticity sufficient for compatibility with a prosthesis rear surface and skin. This allows the film to cater for skin and prosthesis movement and helps to ensure that the prosthesis remains attached.

Preferably, the film is semi-permeable for breathing of skin, thus allowing absorption of exudate and providing user comfort generally.

The front surface adhesive may be an acrylate adhesive. This has been found to be particularly suitable.

In one embodiment, there is selectively incomplete coverage of adhesive on the film rear surface to help allow natural reltive movement between the prosthesis and skin. This helps to allow flexibility of the film to improve attachment effectiveness.

The adhesive may be applied in a regular pattern on the film rear surface. This is preferably a grid pattern, which may extend diagonally in rear view. It has been found that such an arrangement provides good flexibility and user comfort.

In one embodiment, there is selectively incomplete coverage of adhesive on the film front surface to help allow natural relative movement between the prosthesis and skin. This may be in a striped pattern, which may extend transversely across the film in front view. This arrangement helps to prevent damage to the prosthesis and allows expansion and flexibility while still providing the necessary shear strength.

In another embodiment, the film has an arcuate upper side edge so that the fastener conforms approximately to the upper portion of the rear surface of a prosthesis. This arrangement helps to ensure that the prosthesis does not separate from the body, in use.

According to another aspect, the invention provides a method of attaching a breast prosthesis to the skin of a user, the method comprising the steps in any sequence of:-
adhering a film at a rear side thereof to the skin by use of adhesive, and
adhering a prosthesis to a front surface of the film by use of adhesive.

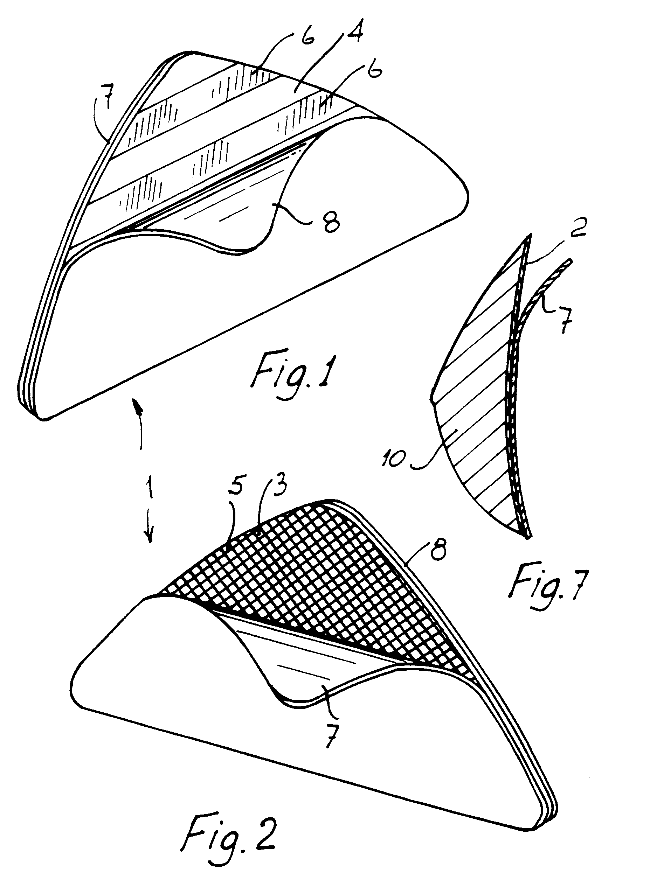

The invention will be more clearly understood from the following description of some embodiments thereof, given by way of example only with reference to the accompanying drawings in which:-
Figs. 1 and 2 are perspective front and rear views respectively of a fastener of the invention;

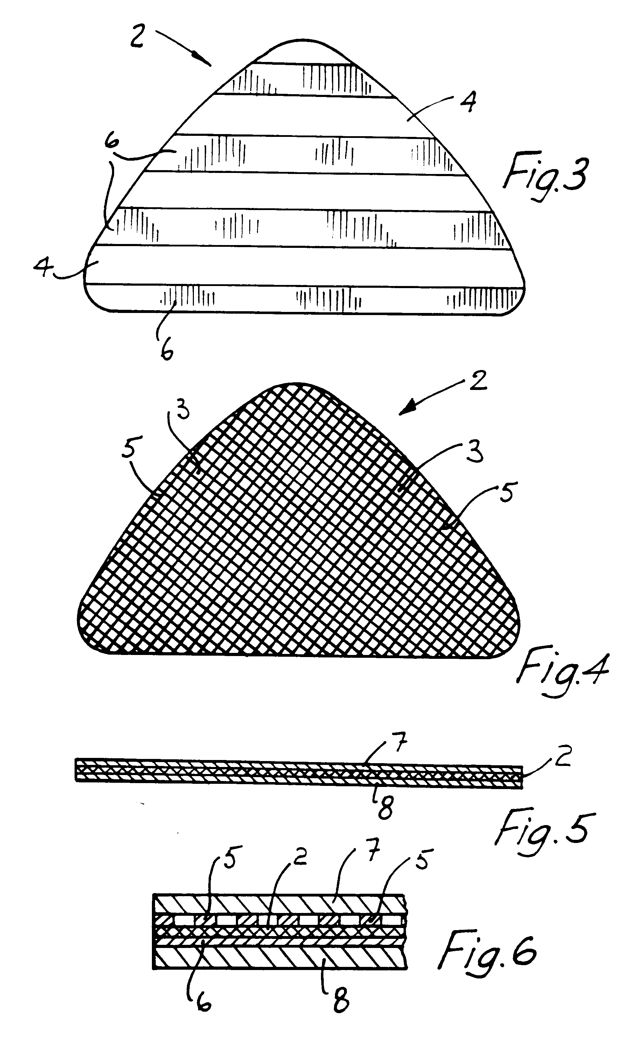

Figs. 3 and 4 are front and rear views of a film of the fastener;
Fig. 5 is a cross-sectional view of the fastener, and
Fig. 6 is a more detailed cross-sectional view showing the adhesives and other parts in more detail;
Fig. 7 is a diagrammatic view showing the film being peeled from a prosthesis, and
Figs. 8(a) to 8(d) are diagrammatic front views showing different possible fastener shapes.

### Detailed Description of the Invention

Referring to the drawings, a fastener of the invention, indicated generally by the reference numeral 1 is shown. The fastener 1 comprises a flexible elastic sheet or film 2 having a rear skin-engaging surface 3 and a front prosthesis-engaging surface 4. The film 2 is approximately 0.9 mm thick and comprises a pair of adhered membranes, namely, a Spyroflex™ Semipermeable Wound Dressing at the rear, and a Catalog No. 9920, 3M™ Fastener Tape Membrane at the front. These membranes have similar elastic and permeability properties. Thus, at the rear, the film 2 comprises a graduated polymer membrane suitable for wound application. The film 2 thus provides the absorptive and transpiration properties which are required for exudate because it comprises small inter-communicating voids which allow absorption of a quantity of exudate and results in the film 2 being semi-permeable. At the rear surface 3, the film membrane is condensed polyurethane which forms a microporous skin-like surface which acts as a semi-permeable membrane. The front membrane of the film 2 is a transparent polyethylene film which is compatible with prosthesis envelopes. The film 2 has a specified elasticity of 200% for compatibility with flexibility and elasticity of the skin and prosthesis. In this specification, the term "film" is intended to cover a sheet of material such as plastics of sufficient thickness to provide the necessary structural strength, but generally thin enough so that there is a small gap between the prosthesis and the skin.

A skin-compatible adhesive 5 is applied in a grid pattern to the rear surface 3 of the film 2, the pattern ensuring that the flexibility of the film 2 is not limited by its adhesion to the skin. The grid pattern of the adhesive 5 is a square pattern with the adhesive lines being 0.7-1.0 mm wide with spacings of 1.6 - 2.1 mm. The direction of the grid is substantially diagonal in rear view. The adhesive 5 is of the type used for medical wound dressings and, as stated above, this particular adhesive is that supplied on dressings marketed under the Trade Name "Spyroflex". A very important feature of the adhesive 5 is that it has a high shear strength for bonding of the prosthesis to the skin, while at the same time it has a low peel resistance so that it may be easily peeled off the skin. In addition, the flexibility and elasticity of the film and selectively incomplete adhesive coverage allow natural movement without discomfort.

The front surface 4 of the film 2 has 12 mm wide strips 6 of adhesive at 14 mm spacings, in this case of the hypoallergenic pressure-sensitive acrylate type provided on the 9920 3M Fastener Tape. This adhesive also has a high shear strength and a low peel resistance.

The shear strength of both adhesives is sufficient to comfortably support a prosthesis weight of 1 Kg for a fastener surface of 60 cm² at least. The direction of the strips 6 is substantially transverse in front view.

Because adhesive is applied to less than half of the front surface 4 of the film 2, flexibility of the film 2 is maintained, and further, it is particularly easy to remove the film from the prosthesis without damaging the skin of the prosthesis. This latter feature is also provided by the low peel resistance of the adhesive 6.

Finally, the fastener 1 comprises a pair of liners 7 and 8 on the rear and front surfaces respectively of the film 2. These liners are silicone coated backing sheets of polyethylene film of the type which maintain integrity of the adhesive before use of the fastener 1.

In use, the woman cleans the skin area to which the prosthesis is to be applied and then removes the rear liner 7 from the fastener 1. The fastener 1 is then applied to the skin by pressing from the centre outwardly against the front liner 8. The fastener 1 is preferably left for several minutes to allow the film 2 to warm to body temperature for maximum adhesion.

The woman then takes any desired conventional prosthesis such as the prosthesis 10 shown in Fig. 7. She then removes the front liner 8 from the fastener 1 secured to her skin and places the prosthesis carefully against the fastener 1 and holds it in place for several minutes. By holding in place, the bond between the prosthesis and the fastener 1 is improved.

The woman may then use the prosthesis for approximately twenty hours but possibly for up to three days, if desired. Because the prosthesis is bonded to the skin, the feel and appearance of the prosthesis is very close to that of the natural breast. It is envisaged that this will lead to considerable improvements for the user because she may after a short period of time forget that the prosthesis is being used and go about her daily life with confidence.

When it is desired to remove the prosthesis, the user carefully grips together the side of the prosthesis and an edge of the fastener 1 and carefully peels them away from the skin. Because there is a low peel resistance of the adhesive 5, this is an easy task. Thereafter, the fastener 1 is simply peeled away from the rear surface of the prosthesis 10, as shown in Fig. 7. Alternatively, the prosthesis 10 may be initially peeled away from the fastener 1, leaving the fastener 1 in situ. Then, the user carefully peels the fastener 1 off the skin. The fastener 1 may then be disposed of and a new fastener from a packet of fasteners which is supplied may then be used the next time the prosthesis is required. At the next use the woman may choose a different prosthesis as the fasteners are of a shape which allow use of different types and shapes of protheses.

Referring now to Fig. 8, various different shapes of fastener of the invention are shown. The most important feature is that the fastener can adhere to the top rear surface of the prosthesis both to provide a good bond and to prevent a gap appearing between the prosthesis and the skin at the top of the prosthesis. In Fig. 8(a), there is shown a fastener 20 having a "square" shape with rounded corners, in Fig. 8(b) a fastener 21 having a triangular shape with curved side edges, in Fig. 8(c) a fastener 22 having a triangular shape with rounded corners but relatively straight side edges, and finally in Fig. 8(d) there is shown a fastener 23 having an arcuate shape. It will be appreciated that all of these shapes have an upper arcuate shape for engagement with the upper part of the rear surface of a prosthesis.

In addition, the invention is not limited to the particular type and pattern of adhesive used. Much on-going development work is being carried out on adhesives for use in wound dressings and other medical applications. It will be immediately appreciated by those skilled in the art that different types and patterns of such adhesive may be used. Important features are that on both the rear and front surfaces the adhesives have a high shear strength to hold the prosthesis in place during everyday use, while at the same time having a low peel resistance so that it may be easily removed without pain when being removed from the skin, and without damage to the prosthesis. Further, the selectively incomplete adhesive coverages help to allow breathing of the skin and allow relative movement. Accordingly, problems which would otherwise occur of skin irritation and discomfort generally are avoided.

It will be appreciated that the invention provides an extremely simple solution to the problems which have existed heretofore in attachment of prostheses to the user's skin. Because any conventional prosthesis may be used the costs are minimalised, the only cost to the user being the relatively small cost of buying a packet of fasteners. Because many different conventional prostheses may be used, there is good versatility for the user, and because only the thickness of the film separates the prosthesis from the skin, the manner in which the prosthesis is attached closely resembles the natural situation. This is extremely important from a psychological point of view.

It is envisaged, however, that the fastener or a set of fasteners may be supplied together with a prosthesis as an assembly.

The invention is not limited to the embodiments hereinbefore described, but may be varied in construction and detail.

## Claims

1. A breast prosthesis fastener (1) comprising a body portion, means for adhering to a users skin, and means for attachment to a prosthesis to hold the prosthesis in place, characterised in that,
the body portion comprises a film (2) shaped to conform to at least portion of the rear surface of a breast prosthesis (10), the film (2) having a skin-engaging rear surface (3) and a prosthesis-engaging front surface (4);
the rear surface (3) has a skin-compatible adhesive (5) of high shear strength and low peel resistance;
the front surface (4) has a plastics-compatible adhesive (6) of high shear strength and low peel resistance for removable attachment of the film (2) to the rear surface of the prosthesis (10).

2. A fastener as claimed in claim 1, wherein the film (2) is of elastic material having an elasticity sufficient for compatibility with a prosthesis rear surface and skin.

3. A fastener as claimed in claims 1 or 2, wherein the film (2) is semi-permeable for breathing of skin.

4. A fastener as claimed in any preceding claim, wherein the front surface adhesive is an acrylate adhesive.

5. A fastener as claimed in any preceding claim, wherein there is selectively incomplete coverage of adhesive (5) on the rear surface (3) of the film (2) to help allow natural relative movement between the prosthesis and skin.

6. A fastener as claimed in claim 5, wherein the adhesive (5) is applied in a regular pattern on the rear surface of the film, such as a grid pattern, which may extend diagonally in rear view.

7. A fastener as claimed in any preceding claim, wherein there is selectively incomplete coverage of adhesive (6) on the front surface of the film to help allow natural relative movement between the prosthesis (10) and skin, the coverage possibly being in a striped pattern, which may extend transversely across the fastener, in front view.

8. A fastener as claimed in any preceding, wherein the film (2) has an arcuate upper side edge so that the fastener conforms approximately to the upper portion of the rear surface of a prosthesis.

9. A breast prosthesis assembly comprising a prosthesis (10) and a fastener (1) as claimed in any preceding claim.

10. A method of attaching a breast prosthesis to the skin of a user, the method comprising the steps in any sequence of:-
adhering a film (2) at a rear surface (3) thereof to the skin by use of adhesive (5), and
adhering a prosthesis (10) to a front surface (4) of the film (2) by use of adhesive (6).
